(19) Europäisches Patentamt
European Patent Office
Office européen des brevets

(11) Veröffentlichungsnummer: **0 174 910**
**B1**

(12) **EUROPÄISCHE PATENTSCHRIFT**

(45) Veröffentlichungstag der Patentschrift: **04.04.90**

(21) Anmeldenummer: **85810406.0**

(22) Anmeldetag: **06.09.85**

(51) Int. Cl.$^5$: **C 07 D 207/416,**
C 07 D 207/333,
C 07 D 207/335,
C 07 C 255/61, C 07 C 229/38,
C 07 C 225/10 // A01N43/36

# (54) Verfahren zur Herstellung von 4-Phenyl-pyrrol-derivaten.

(30) Priorität: **12.09.84 CH 4355/84**

(43) Veröffentlichungstag der Anmeldung:
**19.03.86 Patentblatt 86/12**

(45) Bekanntmachung des Hinweises auf die Patenterteilung:
**04.04.90 Patentblatt 90/14**

(84) Benannte Vertragsstaaten:
**AT BE CH DE FR GB IT LI LU NL SE**

(56) Entgegenhaltungen:
**EP-A-0 018 473**
**EP-A-0 111 452**
**DE-A-1 545 831**
**DE-A-2 927 480**
**US-A-3 775 477**

**CHEMICAL ABSTRACTS, Band 78, Nr. 17, 30. April 1973, Seite 460, Spalte 1, Zusammenfassungsnr. 111044x, Columbus, Ohio, US; A.M. VAN LEUSEN et al.: "Chemistry of sulfonylmethyl isocyanides. 6. New and simple synthesis of the pyrrole ring system from Michael acceptors and (p-tolylsulfonyl)methyl isocyanide"**

(73) Patentinhaber: **CIBA-GEIGY AG**
**Klybeckstrasse 141**
**CH-4002 Basel (CH)**

(72) Erfinder: **Martin, Pierre, Dr.**
**Meisenweg 38**
**CH-4310 Rheinfelden (CH)**

(56) Entgegenhaltungen:
**TETRAHEDRON LETTERS, Nr. 17, 1977, Seiten 1469-1472; D.O. CHENG et al.: "Synthesis of substituted porphyrins"**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 174 910 B1

**Beschreibung**

Die vorliegende Erfindung betrifft eine neues Verfahren zur Herstellung von 4-Phenyl-pyrrol-derivaten der Formel I

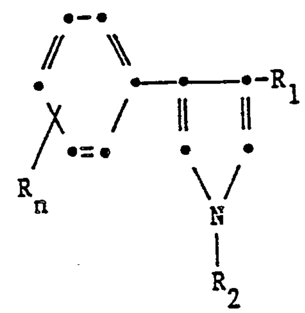

(I)

worin $R_1$ für CN, CHO oder $COOC_1$—$C_6$-Alkyl steht; $R_2$ Wasserstoff, $CH_2CH_2CN$ oder $CH_2CH_2COOC_1$—$C_6$-Alkyl bedeutet; R für Halogen, $C_1$—$C_6$-Alkyl oder $C_1$—$C_6$-Haloalkyl steht; und n für 0, 1 oder 2 steht.

Unter dem Begriff Alkyl selbst als Bestandteil eines anderen Substituenten, wie Haloalkyl etc., sind je nach Zahl der angegebenen Kohlenstoffatome beispielsweise die folgenden geradkettigen oder verzweigten Gruppen zu verstehen: Methyl, Ethyl, Propyl, Butyl, Pentyl, Hexyl usw. sowie ihre Isomeren, wie z.B. Isopropyl, Isobutyl, tert.-Butyl, Isopentyl usw. Die Vorsilbe Halo in der Bezeichnung eines Substituenten bedeutet hier und im folgenden, dass dieser Substituent einfach bis perhalogeniert auftreten kann. Halogen und Halo stehen stellvertretend insbesondere für Fluor, Chlor oder Brom. Haloalkyl steht somit für einen einfach bis perhalogenierten Alkylrest, wie z.B. $CHCl_2$, $CH_2F$, $CCl_3$, $CH_2Cl$, $CHF_2$, $CH_2CH_2Br$, $C_2Cl_5$, CHBr, CHBrCl usw., vorzugsweise für $CF_3$.

4-Phenyl-pyrrol-derivate der Formel I, worin n für 0, 1 oder 2 steht; $R_1$ für Cyano und $R_2$ für Wasserstoff oder Acetyl steht sind aus der Deutschen Offenlegungsschrift 29 27 480[1)] als Pflanzenfungizide bekannt. Verbindungen der Formel I, worin $R_1$ für CHO oder $COOC_1$—$C_6$-Alkyl oder $R_2$ für $CH_2CH_2CN$ oder $CH_2CH_2COOC_1$—$C_6$-Alkyl stehen, können, wie weiter unten gezeigt wird, in einfacher Weise in die bekannten, fungiziden 4-Phenyl-3-cyanopyrröle überführt werden und besitzen somit Zwischenproduktcharakter.

In der Deutschen Offenlegungsschrift 29 27 480[1)] wird ein aus Tetrahedron Letters No. 52, pp 5337—5340, *1972*[2)] bekanntes Verfahren zur Herstellung von 4-Phenyl-3-cyanopyrrol-derivaten angegeben. Bei diesem als TosMIC-Verfahren bekannten Prozess wird ein Zimtsäurenitril der Formel X

(X)

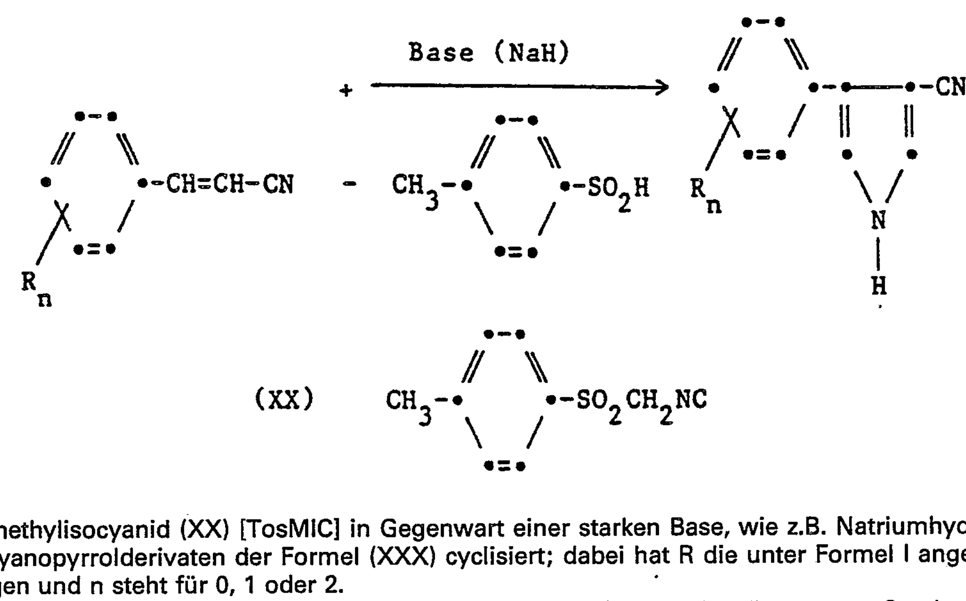


(XXX)

(XX)

mit Tosylmethylisocyanid (XX) [TosMIC] in Gegenwart einer starken Base, wie z.B. Natriumhydrid, zu 4-Phenyl-3-cyanopyrrolderivaten der Formel (XXX) cyclisiert; dabei hat R die unter Formel I angegebenen Bedeutungen und n steht für 0, 1 oder 2.

Obwohl eine Fülle von Pyrrolsynthesen bekannt ist (vgl. J. M. Patterson, Synthesis *1976*, pp. 281—304[3)]), führte bisher lediglich das oben skizzierte TosMIC-Verfahren unmittelbar zu den in 2- und 5-Stellung unsubstituierten, fungizid wertvollen 4-Phenyl-3-cyanopyrrol-derivaten. Allerdings wird in Referenz 2) für die Herstellung des Grundkörpers 4-Phenyl-3-cyanopyrrol die für technische Zwecke geringe Ausbeute von nur 35% angegeben. Es hat sich auch gezeigt, dass das Reagenz TosMIC für technische Synthesen schwerwiegende Nachteile aufweist. So neigt TosMIC bei erhöhten Temperaturen, insbesondere oberhalb von 90°C (übliche Trocknungsbedingungen) zu explosionsartigem Zerfall.

Andererseits verbraucht eine Restfeuchtigkeit einen Teil der eingesetzten Base (Hydrolysegefahr/ Ausbeutereduktion). Ferner zeigt TosMIC physiologische Nachteile wie starke Augen- und Hautirritationen.

Durch die genannten Nachteile erweist sich, das an sich brauchbare Laborverfahren, für die industrielle Produktion von 4-Phenyl-pyrrolderivaten als ungeeignet.

Es wurde nun ein neues, ökonomischeres und umweltfreundlicheres Verfahren gefunden, das mit überraschend hohen Ausbeuten arbeitet.

Das erfindungsgemässe neue Verfahren zur Herstellung der eingangs definierten 4-Phenyl-pyrrolderivate der Formel I besteht darin, dass man ein Phenacylamin der Formel II

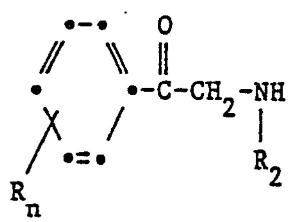

(II)

in Form seines Säureadditionssalzes mit einer Verbindung der Formel III

$$T—CH{=}CH—R_1 \qquad (III)$$

zu einem Zwischenprodukt der Formel IV

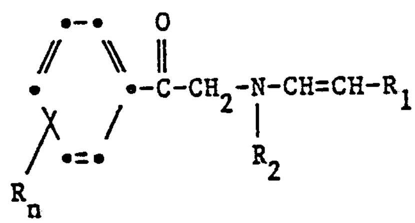

(IV)

umsetzt und dieses in Gegenwart einer Base zu einer Verbindung der Formel I cyclisiert, wobei die Substituenten $R_1$, $R_2$ und $R_n$ in den Formeln II, III und IV die unter Formel I angegebenen Bedeutungen haben, T eine der Gruppe $—OZ$, $—N(R_3)(R_4)$, $—OCOR_a$, $—OSO_2R_b$, $—SR_c$ oder Halogen repräsentiert, wobei Z für $C_1—C_6$-Alkyl, gegebenenfalls substituiertes Phenyl, ein Alkali- oder Erdalkaliatom steht, $R_a$ und $R_b$ unabhängig voneinander für $C_1—C_6$-Alkyl oder gegebenenfalls substituiertes Phenyl stehen, $R_c$ für $C_1—C_3$-Alkyl, $C_1—C_3$-Haloalkyl oder gegebenenfalls substituiertes Phenyl steht; und $R_3$ und $R_4$ unabhängig voneinander für $C_1—C_6$-Alkyl stehen oder gemeinsam mit dem Aminstickstoff einen gesättigten fünf- oder sechsgliedrigen Heterocyclus bilden, der als Heteroatom entweder nur den Aminstickstoff oder ein weiteres Heteroatom enthält.

Eine als gegebenenfalls substituiertes Phenyl bezeichnete Gruppe steht insbesondere für Phenyl oder in para-Position durch Halogen, vorzugsweise Chlor oder Brom, sowie durch $C_1—C_3$-Alkyl, vorzugsweise Methyl substituiertes Phenyl. Als Alkali- bzw. Erdalkaliatome werden z.B. Li, Na und K, vorzugsweise Na und K bzw. Mg, Ca, Sr und Ba, vorzugsweise Mg, Ca und Ba bezeichnet. Steht die Gruppe $—N(R_3)(R_4)$ für einen gesättigten fünf- oder sechsgliedrigen Heterocyclus mit N als Heteroatom oder einem weiteren Heteratom, so sind darunter z.B. die Heterocyclen: Pyrrolidin, Piperazin, Piperidin, Perhydrothiazin, Morpholin, Piperazin, Oxazolidin, Thiazolidin, Imidazolidin, Pyrazolin usw. zu verstehen. Als weiteres Heteratom werden vorzugsweise N, O und S bezeichnet.

Bei dem angegebenen Verfahren ist es nicht notwendig, dass man das Zwischenprodukt (IV) zuerst isoliert und dann zu Verbindungen der Formel I cyclisiert; vielmehr kann die Umsetzung von (II) mit (III) in Gegenwart der Base auch im sogenannten Eintopfverfahren direkt zu den Endprodukten durchgeführt werden, wobei die Zwischenstufe IV nur intermediär durchlaufen wird. Andererseits kann es zweckmässig sein, das Zwischenprodukt IV zuerst, z.B. durch wiederholte Umkristallisation, in besonders reiner Form herzustellen und dann zu einer Verbindung der Formel I zu cyclisieren. Eine bevorzugte Ausführungsform besteht daher in der direkten Umsetzung des Phenylacylamins II in Form seines Säureadditionssalzes in Gegenwart einer Base mit einer Verbindung der Formel III zum Endprodukt I.

Bei der zweiten bevorzugten Ausführungsform wird das Phenacylamin II in Form seines Säureadditionssalzes zunächst in Abwesenheit der Base zu dem Zwischenprodukt IV umgesetzt und (IV) dann erst durch basische Cyclisierung in (I) übergeführt.

Die Reaktionspartner (II), (III) und gegebenenfalls (IV) werden zweckmässigerweise in äquimolaren Mengen eingesetzt; die Base wird vorzugsweise äquimolar oder im Ueberschuss zugegeben.

Typische Vertreter der Verbindungen der Formel III sind die nachfolgend, nicht abschliessend genannten Verbindungen a) bis t), von denen sich die Substanzen a) bis l) als besonders vorteilhaft erwiesen und daher bevorzugt sind:

a) $(CH_3)_2N-CH=CH-CN$

b) $(C_2H_5)_2N-CH=CH-CN$

c) 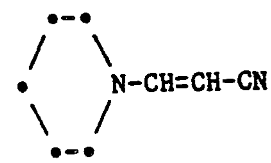

d) 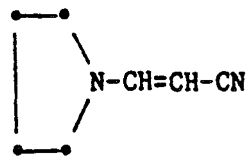

e) 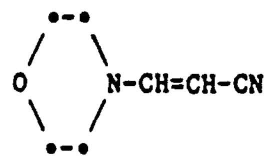

f) $NaO-CH=CH-CN$

g) $KO-CH=CH-CN$

h) $(CH_3)_2N-CH=CH-COOCH_3$

i) $(C_2H_5)_2N-CH=CH-COOCH_3$

k) $(CH_3)_2N-CH=CH-CHO$

l) $(C_2H_5)_2$ $N-CH=CH-CHO$

m) $Cl-CH=CH-CN$

n) $Cl-CH=CH-COOCH_3$

o) $CH_3O_2SO-CH=CH-CN$

p) $[C_6H_4CH_3(4)]-CH=CH-CN$

q) $CH_3O-CH=CH-CN$

r) $C_2H_5O-CH=CH-CN$

s) $C_3H_7O-CH=CH-CN$

t) $[C_6H_4Cl(4)]O-CH=CH-COOCH_3$

Das erfindungsgemässe Verfahren wird zweckmässigerweise in einem reaktionsinerten Lösungsmittel oder Lösungsmittelgemisch durchgeführt, so können ein oder mehrere reaktionsinerte Lösungs- oder Verdünnungsmittel anwesend sein. In Frage kommen beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon; Alkohole, vor allem Alkanole, wie z.B. Methanol, Ethanol, Propanole, Butanole usw. sowie Wasser und wässrige Zweiphasengemische und Gemische der genannten Lösungsmittel untereinander. Für die organische, mit Wasser nicht mischbare Phase kommen dabei z.B. folgende Lösungsmittel in Frage: aliphatische und aromatische Kohlenwasserstoffe, wie Pentan, Xylole usw., halogenierte Kohlenwasserstoffe, wie Dichlormethan, Chloroform, Tetrachlorkohlenstoff, Ethylendichlorid, 1,2-Dichlorethan, Tetrachlorethylen usw. oder aliphatische Ether, wie Diethylether, Diisopropylether, t-Butylmethylether usw. Dabei kann sich die Zugabe eines Phasentransferkatalysators als vorteilhaft erweisen. Beispiele geeigneter Phasentransfer-Katalystoren sind: Tetraalkylammoniumhalogenide, -hydrogensulfate oder -hydroxide wie Tetrabutylammoniumchlorid, -bromid, -jodid; Triethylbenzylammoniumchlorid, -bromid; Tetrapropylammoniumchlorid, -bromid, -jodid; usw. Als Phasentransfer-Katalystoren kommen ferner Phosphonium-Salze in

Betracht. Andererseits wirkt das Ammoniumsalz der Formel II bereits selbst als Phasentransferkatalysator.

Besonders geeignete Lösungsmittel sind Nitrile und Niederalkanole, insbesondere Acetonitril und Ethanol, sowie Alkanol-Wasser-Gemische (Ethanol/$H_2O$).

Die Reaktionstemperaturen liegen in der Regel bei allen Teilschritten und im Eintopfverfahren zwischen 0° und +120°C, vorzugsweise bei +30° bis +80°C.

Die Verbindung der Formel II wird erfindungsgemäss, auf Grund der verminderten thermischen Stabilität des zugrundeliegenden Phenacylamins, in Form ihres stabileren Ammoniumsalzes eingesetzt, das durch übliche Addition einer organischen oder anorganischen Säure an das freie Amin erhältlich ist.

Beispiele salzbildender Säuren sind anorganische Säuren: Halogenwasserstoffsäuren wie Fluorwasserstoffsäure, Chlorwasserstoffsäure, Bromwasserstoffsäure oder Jodwasserstoffsäure sowie Schwefelsäure, Phosphorsäure, Phosphorige Säure, Salpetersäure usw. sowie organische Säuren wie Essigsäure, Trifluoressigsäure, Trichloressigsäure, Propionsäure, Glycolsäure, Milchsäure, Bernsteinsäure, Benzoesäure, Zimtsäure, Oxalsäure, Ameisensäure, Benzolsulfonsäure, p-Toluolsulfonsäure, Methansulfonsäure, Salicylsäure, 2-Phenoxybenzoesäure oder 2-Acetoxybenzoesäure usw.

Bevorzugt sind starke Säuren wie z.B. die Halogenwasserstoffsäuren, die Phosphorsäure, Salpetersäure, sowie die Sulfonsäuren, wie die p-Toluolsulfonsäure. Besonders bevorzugt ist die Salzsäure.

Die Umsetzung von (II) mit (III) direkt zu (I) oder von (IV) zu (I) erfolgt erfindungsgemäss in Gegenwart einer Base. Geeignete Basen sind beispielsweise anorganische Basen, wie die Oxide, Hydride, Hydroxide, Carbonate, Carbonsäuresalze und Alkoholate der Erdalkali-, bevorzugt der Alkalimetalle, insbesondere die des Natriums und Kaliums [z.B. NaH, NaOH, KOH, $Na_2CO_3$, $K_2CO_3$, $CaCO_3$, $CH_3COONa$, $C_2H_5COOK$, $C_2H_5ONa$, $CH_3ONa$, usw.], bevorzugt die Alkalialkoholate wie Natriumethylat oder Natriummethylat. Als organische Basen eignen sich Trialkylamine wie z.B. Triethylamin, Piperidin, Pyridin, 4-Dimethylaminopyridin, usw.

Bei den erfindungsgemässen Herstellungsverfahren können anfallende Zwischen- und End-Produkte aus dem Reaktionsmedium isoliert und, falls gewünscht, auf eine der allgemein üblichen Methoden gereinigt werden, z.B. durch Extraktion, Kristallisation, Chromatographie, Destillation usw. Man kann die Herstellung der Verbindungen der Formel I jedoch im allgemeinen auch mit guten Ausbeuten und hoher Reinheit in der genannten Eintopfreaktion durchführen und auf eine Isoslierung von Zwischenprodukten grundsätzlich verzichten.

Bevorzugte Ausführungsformen des erfindungsgemässen Verfahrens sind z.B. diejenigen, bei denen:

a) Ausgangsprodukte der Formel II eingesetzt werden, die sich dadurch kennzeichnen, dass R für Halogen, vorzugsweise für Fluor, Chlor oder Brom, insbesondere für Chlor steht; n für 1 oder vorzugsweise 2 steht, wobei im Falle von n = 2 insbesondere die ortho- und meta-Stellung bevorzugt ist, $R_1$ für CN steht und $R_2$ für Wasserstoff steht.

b) Reagenzien der Formel III eingesetzt werden, die sich dadurch kennzeichnet, dass T für eine der Gruppen

$-N(CH_3)_2$, $-N(C_2H_5)_2$, 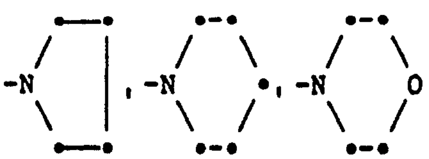 , -OK oder -ONa steht und

$R_1$ CN, $COOCH_3$ oder CHO, vorzugsweise CN repräsentiert.

c) Zwischenprodukte der Formel IV eingesetzt werden, die sich dadurch kennzeichnen, dass $R_1$, $R_2$ und $R_n$ die unter a) und b) angegebenen Bedeutungen haben.

d) Säureadditionssalze der Formel II einsetzt, die sich dadurch kennzeichnen, dass sie als Säurekomponente eine Halogenwasserstoffsäure, vorzugsweise Salzsäure; eine Sulfonsäure, vorzugsweise Benzol- oder p-Toluolsulfonsäure oder Schwefelsäure enthalten.

e) man die Reaktion von (II) mit (III) so führt, dass das Zwischenprodukt IV nur intermediär auftritt.

f) man im Temperaturbereich zwischen +30° und +80°C arbeitet.

Eine besonders bevorzugte Ausführungsform besteht somit darin, dass man 2,3-Dichlorphenacylamin in Form seines Säureadditionssalzes, vorzugsweise in Form seines Hydrochlorids, mit einer Verbindung der Formel III, worin $R_1$ für CN steht und T eine der Gruppen

$-N(CH_3)_2$, $-N(C_2H_5)_2$, 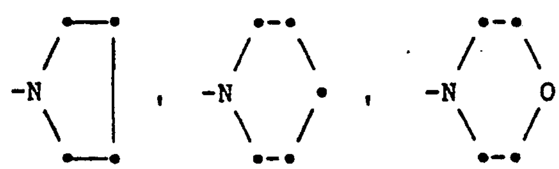 , -OK oder -ONa,

vorzugsweise $-N(CH_3)_2$, 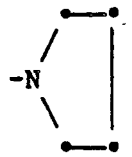 , 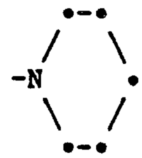 oder 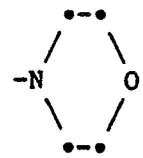

repräsentiert, zu 3-(2,3-Dichlorphenacylamino)-acrylonitril umsetzt und letzteres als Substanz oder vorzugsweise in situ in Gegenwart einer Base, vorzugsweise in Gegenwart eines Niederalkanolates, Natronlauge, Kalilauge, Natriumacetat, Kaliumacetat oder eines Triniederalkylamins zu 4-(2,3-Dichlorphenyl)-3-cyanopyrrol cyclisiert.

Die Ausgangsprodukte der Formel II sind grösstenteils bekannt oder können analog zu den bekannten Vertretern hergestellt werden. Neu hingegen ist 2,3-Dichlorphenacylamin und seine Säureadditionssalze. Diese Substanz ist auf Grund ihrer Struktur als Zwischenprodukt für die erfindungsgemässe Herstellung des fungizid aktiven 4-(2,3-Dichlorphenyl)-3-cyanopyrrols prädestiniert und daher ein Bestandteil dieser Erfindung. Ihre Herstellung wird weiter unten explizite beschrieben.

Verbindungen der Formel II, worin $R_2$ für $CH_2CH_2CN$ oder $CH_2CH_2COO—C_1—C_6$-Alkyl steht lassen sich aus den zugrundeliegenden Phenacylaminen II (mit $R_2 =$ H) z.B. wie folgt herstellen: Das Säureadditionssalz (z.B. HCl-Salz) eines am Stickstoff unsubstituierten Phenacylamins der Formel II wird in Gegenwart einer äquimolaren Menge von Acrylonitril bzw. eines Acrylsäure-$C_1—C_6$-alkylesters, vorzugsweise in Gegenwart einer der eingangs genannten Basen und unter den für die Reaktion von (II) mit (III) zu (I) genannten Bedingungen umgesetzt.

Typische Vertreter von Verbindungen der Formel II sind im Rahmen der vorliegenden Erfindung beispielsweise die in Tabelle 1 genannten Substanzen.

Tabelle 1: Verbindungen der Formel II

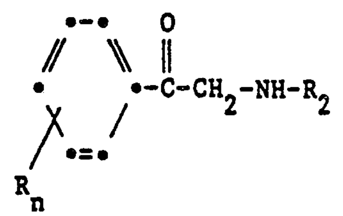 . (II)

| Verb. Nr. | $R_n$ | $R_2$ |
|---|---|---|
| 1.1 | H | H |
| 1.2 | 3-Cl | H |
| 1.3 | $2,4-Cl_2$ | H |
| 1.4 | 4-Cl | H |
| 1.5 | 4-F | H |
| 1.6 | $3-CH_3$ | H |
| 1.7 | 3-F | H |
| 1.8 | 3-Br | H |
| 1.9 | $3-CF_3$ | H |
| 1.10 | 2-Cl | H |
| 1.11 | $2,3-Cl_2$ | H |

Tabelle 1 (Fortsetzung)

| Verb. Nr. | $R_n$ | $R_2$ |
|---|---|---|
| 1.12 | 2,5-$Cl_2$ | H |
| 1.13 | 2-Br | H |
| 1.14 | 2,6-$Cl_2$ | H |
| 1.15 | H | $CH_2CH_2CN$ |
| 1.16 | 3-Cl | $CH_2CH_2CN$ |
| 1.17 | 2-Cl | $CH_2CH_2CN$ |
| 1.18 | 2,3-$Cl_2$ | $CH_2CH_2CN$ |
| 1.19 | 3-F | $CH_2CH_2CN$ |
| 1.20 | 3-Cl | $CH_2CH_2COOCH_3$ |
| 1.21 | 2,3-$Cl_2$ | $CH_2CH_2COOCH_3$ |
| 1.22 | 2-Cl | $CH_2CH_2COOCH_3$ |
| 1.23 | 2,3-$Cl_2$ | $CH_2CH_2COOC_2H_5$ |
| 1.24 | 2,3-$Cl_2$ | $CH_2CH_2COOC_3H_7$ |
| 1.25 | 2-Br | $CH_2CH_2COOCH_3$ |

Bei den Verbindungen der Formel III handelt es sich im allgemeinen um käufliche und somit bekannte Substanzen oder um Verbindungen, die analog zu ihren bekannten Vertretern hergestellt werden können.

Die Herstellung der Zwischenprodukte der Formel IV stellt einen Teil des erfindungsgemässen Verfahrens dar und wurde weiter oben detailliert beschrieben. Diese Zwischenprodukte IV lassen sich durch einfache, basische Cyclisierung in die wertvollen Fungizide der Formel I transformieren, zeigen bereits ihrerseits fungizide Aktivität und stellen somit einen wesentlichen Bestandteil dieser Erfindung dar.

Typische Verteter von Zwischenprodukten der Formel IV sind im Rahmen vorliegender Erfindung:

Tabelle 2: Verbindungen der Formel

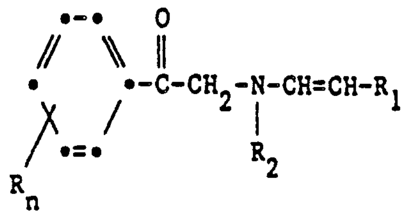

(IV)

| Verb. Nr. | $R_n$ | $R_2$ | $R_1$ |
|---|---|---|---|
| 2.1 | H | H | CN |
| 2.2 | 3-Cl | H | CN |
| 2.3 | 2,4-Cl | H | CN |
| 2.4 | 4-Cl | H | CN |
| 2.5 | 4-F | H | CN |
| 2.6 | $3-CH_3$ | H | CN |
| 2.7 | 3-F | H | CN |
| 2.8 | 3-Br | H | CN |
| 2.9 | $3-CF_3$ | H | CN |
| 2.10 | 2-Cl | H | CN |
| 2.11 | $2,3-Cl_2$ | H | CN |
| 2.12 | $2,5-Cl_2$ | H | CN |
| 2.13 | 2-Br | H | CN |
| 2.14 | $2,6-Cl_2$ | H | CN |
| 2.15 | $2,3-Cl_2$ | H | $COOCH_3$ |
| 2.16 | H | H | CHO |
| 2.17 | 3-Cl | H | $COOCH_3$ |
| 2.18 | $3,4-Cl_2$ | H | $COOCH_3$ |

Tabelle 2: (Fortsetzung)

| Verb. Nr. | $R_n$ | $R_2$ | $R_1$ |
|---|---|---|---|
| 2.19 | 2-Cl | H | $COOCH_3$ |
| 2.20 | 2,3-$Cl_2$ | H | $COOC_3H_7$ |
| 2.21 | 2,3-$Cl_2$ | $CH_2CH_2COOCH_3$ | CN |
| 2.22 | 2,3-$Cl_2$ | $CH_2CH_2CN$ | CN |
| 2.23 | H | $CH_2CH_2CN$ | CN |
| 2.24 | 3-Cl | $CH_2CH_2CN$ | CN |
| 2.25 | 2-Cl | $CH_2CH_2CN$ | CN |
| 2.26 | 2,3-$Cl_2$ | $CH_2CH_2COOC_2H_5$ | CN |
| 2.27 | 3-F | $CH_2CH_2CN$ | CN |
| 2.28 | 3-Cl | $CH_2CH_2COOCH_3$ | CN |
| 2.29 | 2-Cl | $CH_2CH_2COOCH_3$ | CN |
| 2.30 | 2,3-$Cl_2$ | $CH_2CH_2COOC_3H_7$ | CN |
| 2.31 | 2-Br | $CH_2CH_2COOCH_3$ | CN |
| 2.32 | 2,3-$Cl_2$ | $CH_2CH_2CN$ | CHO |

Wie bereits eingangs erwähnt, besitzt ein Teil der Verbindungen der Formel I Zwischenprodukt-Charakter. Es handelt sich hierbei um die von jetzt an als Untergruppe Ia bezeichneten Vertreter der Formel I, die sich dadurch kennzeichnen, dass $R_n$ die unter Formel I angegebenen Bedeutungen hat; und in den Fällen, in denen $R_1$ für Formyl oder $C_2$—$C_6$-Alkoxycarbonyl steht, $R_2$ gleichzeitig für Wasserstoff, $CH_2CH_2CN$ oder $CH_2CH_2COOC_1$—$C_6$-Alkyl steht, oder in den Fällen, in denen $R_1$ für CN steht, $R_2$ gleichzeitig $CH_2CH_2CN$ oder $CH_2CH_2COOC_1$—$C_6$-Alkyl bedeutet. Diese neuen, fungizid ebenfalls aktiven Pyrrolderivate lassen sich in einfacher Weise in die aus der DE—2927480 bekannten, fungiziden 4-Phenyl-3-cyanopyrrole umwandeln, da CHO und $COOC_1$—$C_6$-Alkyl in CN unwandelbar sind und $CH_2CH_2CN$ sowie $CH_2CH_2COOC_1$—$C_6$-Alkyl als Substituenten am Pyrrolstickstoffatom leicht abspaltbare Gruppen darstellen.

Typische Beispiele von Verbindungen der Untergruppe Ia werden nachfolgend genannt.

Tabelle 3: Verbindungen der Formel Ia

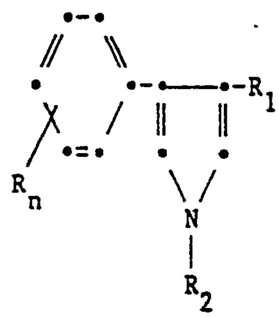

(Ia)

| Verb. Nr. | $R_n$ | $R_1$ | $R_2$ |
|---|---|---|---|
| 3.1 | H | CHO | H |
| 3.2 | 3-Cl | CHO | H |
| 3.3 | 2,4-$Cl_2$ | CHO | H |
| 3.4 | 4-$Cl_2$ | CHO | H |
| 3.5 | 4-F | CHO | H |
| 3.6 | 3-$CH_3$ | CHO | H |
| 3.7 | 3-F | CHO | H |
| 3.8 | 3-$CF_3$ | CHO | H |
| 3.9 | 2,3-$Cl_2$ | CHO | H |
| 3.10 | 2,6-$Cl_2$ | CHO | H |
| 3.12 | 3-Cl | $COOCH_3$ | H |
| 3.13 | 2-Cl | $COOCH_3$ | H |

Tabelle 3 (Fortsetzung)

| Verb. Nr. | $R_n$ | $R_1$ | $R_2$ |
|---|---|---|---|
| 3.14 | 4-F | $COOCH_3$ | H |
| 3.15 | 2,3-$Cl_2$ | $COOCH_3$ | H |
| 3.16 | 3-Cl | CN | $CH_2CH_2CN$ |
| 3.17 | 2-Cl | CN | $CH_2CH_2CN$ |
| 3.18 | 3-$CH_3$ | CN | $CH_2CH_2CN$ |
| 3.19 | 2,3-$Cl_2$ | CN | $CH_2CH_2CN$ |
| 3.20 | 4-F | CN | $CH_2CH_2COOCH_3$ |
| 3.21 | 2-Cl | CN | $CH_2CH_2COOCH_3$ |
| 3.22 | 2,3-$Cl_2$ | CN | $CH_2CH_2COOCH_3$ |
| 3.23 | 2,3-$Cl_2$ | CHO | $CH_2CH_2COOCH_3$ |
| 3.24 | 2,3-$Cl_2$ | $COOCH_3$ | $CH_2CH_2CN$ |

Die Umwandlung von CHO in CN kann, in an sich bekannter Weise, z.B. folgendermassen erfolgen: Ein Aldehyd der Formel I ($R_1$ = CHO) wird bei 0° bis 100°C in einem reaktionsinerten Lösungsmittel (z.B. Alkohol, Ether, Pyridin, Triethylamin, usw.) mit Hydroxylamin-Hydrochlorid in Gegenwart einer Base (z.B. Natriumacetat, $NaHCO_3$, Pyridin, Triethylamin, usw.) in das entsprechende Oxim (syn/anti-Gemisch) übergeführt und das resultierende Oxim durch Behandlung mit einem wasserentziehenden Mittel (z.B. Essigsäureanhydrid, Cyanurchlorid/Pyridin, $(PNCl_2)_3$, Dicyclohexylcarbodiimid/$CuCl_2$/Triethylamin, $P_2O_5$, Tosylchlorid/Pyridin, $TiCl_4$/Pyridin, usw.) in das Nitril übergeführt.

Soll die Estergruppe $COOC_1$—$C_6$-Alkyl in die CN-Gruppe übergeführt werden, so geht man z.B. am günstigsten von der freien Säure aus, die in an sich bekannter Weise durch Esterhydrolyse mit einer wässrigen Mineralsäure (z.B. $HCl/H_2O$) in Gegenwart eines Lösungsvermittlers (wie Alkohol, Dioxan, Tetrahydrofuran, usw.) am zweckmässigsten unter Rückflusstemperatur hergestellt wird. Die freie Säure wird dann entweder direkt mit Ammoniak bei erhöhter Temperatur oder über ihr Säurechlorid (—COOH + Thionylchlorid → —COCl) mit Ammoniak bei Raumtemperatur in das Säureamid übergeführt und letzteres mit einem der bereits genannten wasserentziehenden Mittel bei Temperaturen zwischen 80° und 220°C zum Nitril verwandelt.

Soll das freie Pyrrol durch Abspaltung des Restes $CH_2CH_2CN$ oder $CH_2CH_2COOC_1$—$C_6$-Alkyl gebildet werden, so kann dies z.B. durch Behandeln mit einer Base bei −20° bis +180°C in einem geeigneten, reaktionsinerten Lösungsmittel erfolgen. Als Beispiele geeigneter Reaktionsbedingungen seien genannt:

a) Natriumhydrid in Dimethylformamid bei 0°C
b) Ammoniak/Wasser/Dioxan bei 180°C
c) Kalilauge/Wasser/Alkohol bei 100°C.

## Herstellungsbeispiele

### Beispiel H1

Herstellung von

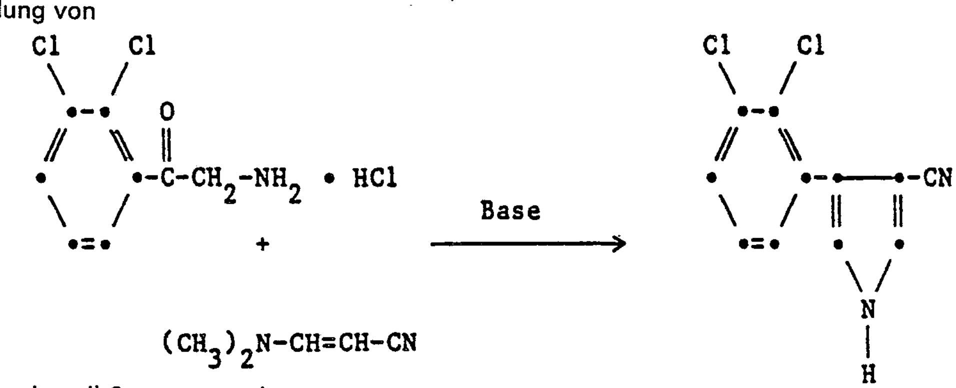


4-(2,3-Dichlorphenyl)-3-cyanopyrrol

a) *Herstellung der Vorstufe N-Acetyl-2,3-dichlorphenacylamin*

150 g 2,3-Dichlorbenzoylcyanid werden bei Normaldruck und bei 70°C in 1,5 Liter Eisessig und 84,15 g Acetanhydrid über 5 g $PtO_2$ mit elementarem Wasserstoff hydriert. Nach Aufnahme von 112% der berechneten Wasserstoffmenge (nach ca. 5 Stunden) wird die Hydrierung abgebrochen, das Reaktionsgemisch filtriert und eingedampft. Das resultierende gelbe Oel wird durch Zugabe von Hexan/Diethylether zur Kristallisation gebracht. Das kristalline Produkt wird abfiltriert und getrocknet. Smp. 107—109°C. IR (fest/KBr) in $cm^{-1}$: 3300 (NH); 1735 (CO); 1650 (CO). $^1H$—NMR ($CDCl_3$) in ppm: 2,08 (s, 3H); 4,55 (d, 2H); 6,2—6,6 (breites s, 1H); 7.25 (m, 3H).

b) *Herstellung der Vorstufe 2,3-Dichlorphenacylamin-Hydrochlorid*

50,0 g des nach a) hergestellten N-Acetyl-2,3-dichlorphenacylamins werden in 500 ml 2N Salzsäure 2 Stunden unter Rückfluss erhitzt. Nach dem Eindampfen der leicht trüben Reaktionslösung wird der Rückstand mit Ethylacetat digeriert. Das kristalline 2,3-Dichlorphenacylamin-Hydrochlorid wird abfiltriert und getrocknet. Smp. 217—218°C. IR (fest/KBr) in $cm^{-1}$: 1695 (CO); (Eine andere Kristallmodifikation zeigt zwei Carbonylschwingungsbanden bei 1690 und 1705 $cm^{-1}$). $^1H$—NMR (DMSO, d6) in ppm: 4,54 (s, 2H); 7,6 (t, 1H); 7,9 (m, 2H); 8,6 (s, 3H, mit $D_2O$ austauschbar).

c) *Herstellung des Endprodukts 4-(2,3-Dichlorphenyl)-3-cyanopyrrol*

20,0 g 2,3-Dichlorphenacylamin-Hydrochlorid und 10,0 g 3-Dimethylaminoacrylonitril werden in 300 ml Ethanol 1 Stunde unter Rückfluss erhitzt. Dann lässt man eine ethanolische Lösung von Natriumethylat, hergestellt aus 2,1 g Natrium und 30 ml Ethanol, rasch zutropfen und erhitzt das Reaktionsgemisch weitere 10 Minuten unter Rückfluss. Nach Abkühlen auf Raumtemperatur wird das Reaktionsgemisch auf Eis/Salzsäure gegossen und die resultierende Mischung 1,5 Stunden gerührt. Der Niederschlag wird abfiltriert, mit Wasser gewaschen und getrocknet: 15,4 g (78% der Theorie) Smp. 152—154°C.

### Beispiele H2 bis H4

Herstellung von

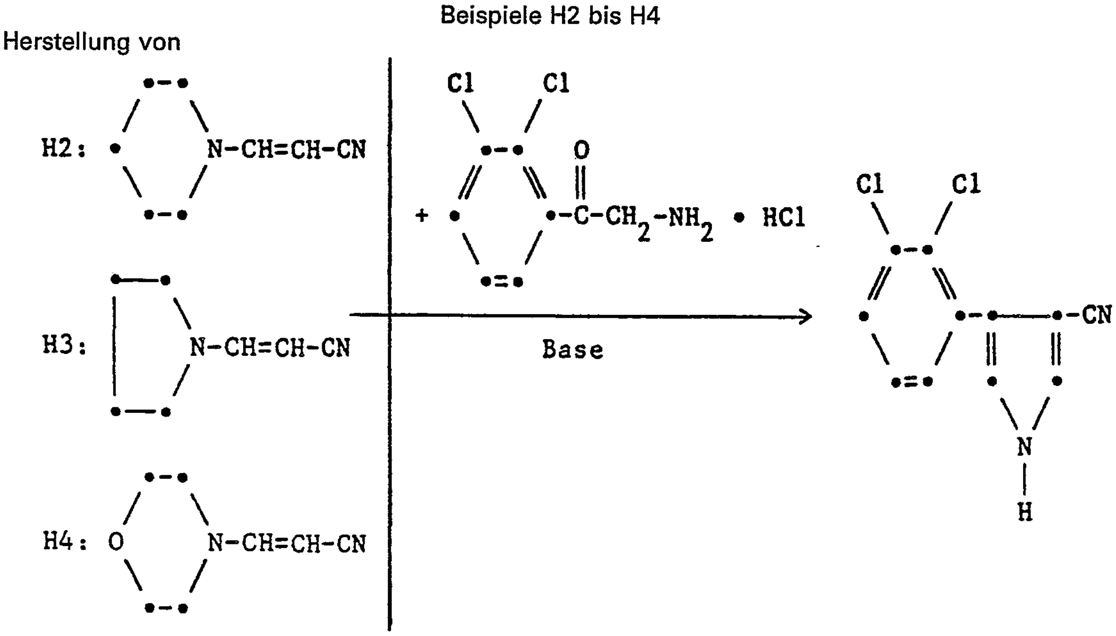

4-(2,3-Dichlorphenyl)-3-cyanopyrrol

Man verfährt wie im Herstellungsbeispiel HIc, ersetzt jedoch 3-Dimethylaminoacrylonitril durch
N-Piperidinylacrylonitril,
N-Pyrrolidinylacrylonitril, oder
N-Morpholinylacrylonitril
und erhöht die Rückflusszeit von 1 Stunde auf 3 bis 4 Stunden. Man erhält in allen 3 Fällen reines 4-(2,3-Dichlorphenyl)-3-cyanopyrrol mit Ausbeuten zwischen 76 und 85% der Theorie. Smp. 150—154°C.

Beispiel H5

Herstellung von

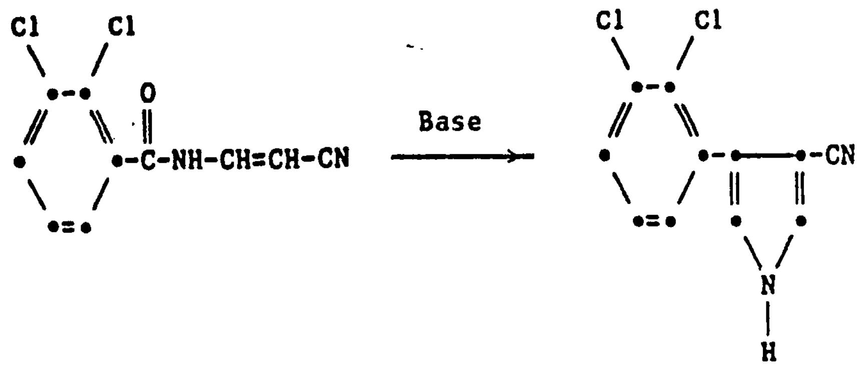


4-(2,3-Dichlorphenyl)-3-cyanopyrrol

a) *Herstellung des Zwischenproduktes 3-(2,3-Dichlorphenacylamino)-acrylonitril*

20,0 g 2,3-Dichlorphenacylamin-Hydrochlorid und 10,0 g 3-Dimethylaminoacrylonitril werden in 300 ml Ethanol 1 Stunde unter Rückfluss erhitzt. Nach Abkühlen auf Raumtemperatur wird die Reaktionslösung auf ein Eis/verdünnte Salzsäure-Gemisch gegossen und mit Essigsäureethylester extrahiert. Die vereinigten Extrakte werden über Natriumsulfat getrocknet, filtriert und eingedampft. Der ölige Rückstand wird säulenchromatographisch (Kieselgel-Toluol/Essigsäureethylester 4:1) gereinigt. Smp. 125—127°C. IR (fest/KBr) in $cm^{-1}$: 3380 (NH), 2200 (CN), 1715 (CO) 1625 (C=C). $^1$H—NMR ($DMSO_{d6}$) in ppm: 4,09 (d, J=15Hz, 1H); 4,46 (d, J=7Hz, 2H); 7,2 (q, 1H); 7,4 (breites s, 1H); 7,45—7,85 (m, 3H). Massenspektrum: Molekül peak bei 254.

b) *Herstellung des Endproduktes 4-(2,3-Dichlorphenyl)-3-cyanopyrrol*

4,2 g des nach a) hergestellten 3-(2,3-Dichlorphenacylamino)-acrylonitrils werden in 50 ml Ethanol mit 0,5 g Natriumethylat versetzt. Das Reaktionsgemisch wird bis zur Rückflusstemperatur erhitzt, auf Raumtemperatur abkühlen gelassen, auf ein Gemisch aus verdünnter Salzsäure und Eis gegeossen und noch ca. 1 Stunde gerührt. Der gebildete Niederschlag wird abfiltriert, mit Wasser gewaschen und getrocknet. Man erhält die Titelsubstanz in quantitativer Ausbeute. Smp. 149—150°C.

Beispiel H6 (Formeln vgl. H5)

a) *Herstellung des Zwischenproduktes 3-(2,3-Dichlorphenacylamino)-acrylonitril*

2 g 2,3-Dichlorphenacylamin-Hydrochlorid, 1 g 3-Hydroxyacrylonitril-Natriumsalz und 20 ml Ethanol werden 2 Stunden unter Rückfluss erhitzt. Das Reaktionsgemisch wird eingedampft und der ölige Rückstand säulenchromatographisch (Kieselgel-Toluol/Essigsäureethylester 4:1) gereinigt. Man erhält das 3-(2,3-Dichlorphenacylamino)-acrylonitril im cis/trans-Verhältnis von 5:1. Smp. 122—125°C.

b) *Herstellung des Endproduktes 4-(2,3-Dichlorphenyl)-3-cyanopyrrol*

4,2 g des nach a) hergestellten 3-(2,3-Dichlorphenacylamino)-acrylonitrils wurden in 50 ml Ethanol mit 0,5 g Natriumethylat gemäss Beispiel H5b umgesetzt. Man erhält die Titelsubstanz in quantitativer Ausbeute. Smp. 150—152°C.

Beispiel H7

Herstellung von

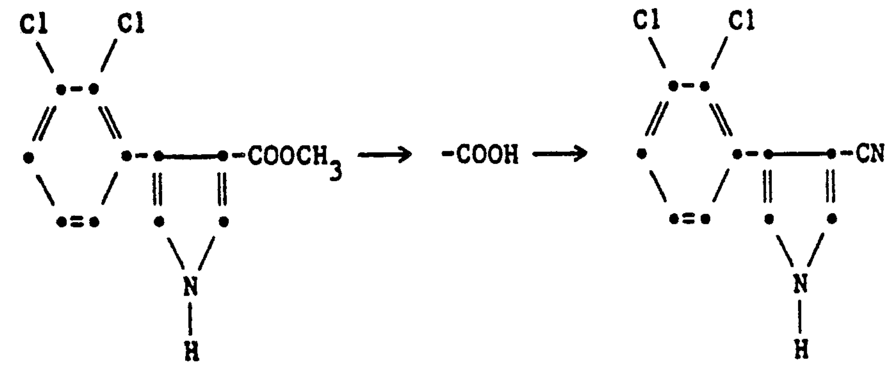

4-(2,3-Dichlorphenyl)-3-cyanopyrrol

a) *Herstellung von 3-Carbomethoxy-4-(2,3-dichlorphenyl)-pyrrol*

10,7 g 2,3-Dichlorphenacylamin-Hydrochlorid und 6 g 3-Dimethylaminoacrylsäuremethylester werden in 120 ml Ethanol 2 Stunden unter Rückfluss erhitzt. Anschliessend lässt man eine Lösung von 4 g Natriumethylat in 40 ml Ethanol zutropfen und erhitzt eine weitere Stunde unter Rückfluss. Dann wird das Reaktionsgemisch eingedampft und der ölige Rückstand säulenchromatographisch (Kieselgel-Toluol/Essigsäureethylester 3:1) gereinigt. Smp. 205—206°C.

b) *Herstellung der Vorstufe 4-(2,3-Dichlorphenyl)pyrrol-3-carbonsäure*

3,2 g des nach a) hergestellten 3-Carbomethoxy-4-(2,3-dichlorphenyl)-pyrrols und 40 ml eines 1:1-Gemisches aus Methanol und 5-normaler HCl werden 5 Stunden bei 70°C gerührt. Nach Abkühlen auf Raumtemperatur wird das Reaktionsgemisch auf Eis gegossen und mit Essigsäureethylester extrahiert. Die Esterphase wird nunmehr ihrerseits mit 10%iger Natronlauge extrahiert. Der wässrige Extrakt wird zweimal mit Essigsäureethylester gewaschen, mit Salzsäure angesäuert und seinerseits erneut mit Essigsäureethylester extrahiert. Die organische Phase wird mit Wasser gewaschen, über Magnesiumsulfat getrocknet, filtriert und eingedampft. Die erhaltene 4-(2,3-Dichlorphenyl)pyrrol-3-carbonsäure schmilzt bei 180—182°C.

c) *Herstellung des Endproduktes 4-(2,3-Dichlorphenyl)-3-cyanopyrrol*

2,1 g der nach b) hergestellten freien 4-(2,3-Dichlorphenyl)pyrrol-3-carbonsäure werden in 30 ml Ethanol gelöst. Die Lösung wird mit konzentriertem Ammoniak alkalisch eingestellt und dann bis zur Trockne eingedampft. Der Rückstand wird erneut in 50 ml Ethanol gelöst. Bei Raumtemperatur werden im Autoklaven 20 atm $NH_3$-Gas auf diese Lösung gegeben und das Reaktionsgemisch 15 Stunden bei 220°C gehalten. Die erkaltete (Raumtemperatur) Reaktionsmischung wird auf Eis/HCl gegossen, der ausgefallene Niederschlag abfiltriert und bei 60°C getrocknet. Das resultierende Pulver wird mit 17 g Polyphosphorsäure im offenen Gefäss auf 180°C erhitzt, das heisse Gemisch tropfenweise auf Eis gegossen, mit NaOH alkalisch eingestellt und mit Essigsäureethylester extrahiert. Die vereinigten Extrakte werden eingedampft und der Rückstand säulenchromatographisch (Kieselgel-Toluol/Essigsäureethylester 4:1) gereinigt. Man erhält 4-(2,3-Dichlorphenyl)-3-cyanopyrrol mit Smp. 148—150°C.

Beispiel H8

Herstellung von

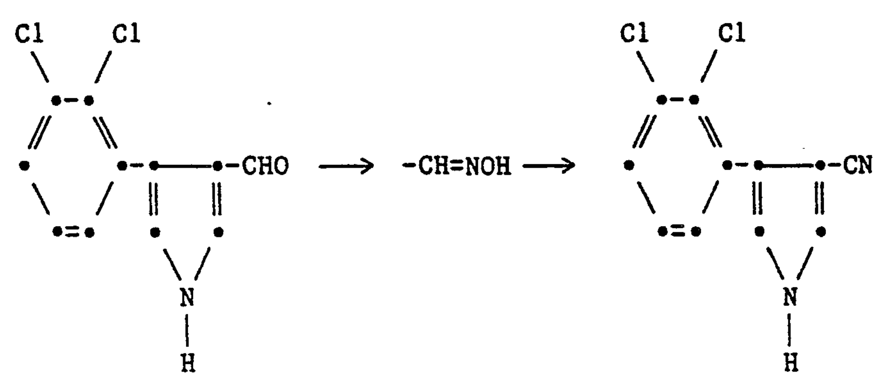

4-(2,3-Dichlorphenyl)-3-cyanopyrrol

a) *Herstellung von 3-Formyl-4-(2,3-dichlorphenyl)pyrrol*

5,4 g 3-Dimethylaminoacrolein, 3,2 g 2,3-Dichlorphenacylamin-Hydrochlorid und 60 ml Ethanol

werden 1,5 Stunden unter Rückfluss erhitzt. Dann wird eine Lösung von Natriumethylat in Ethanol — hergestellt aus 1 g Na und 15 ml Ethanol — zutropfengelassen und das Reaktionsgemisch anschliessend noch weitere 30 Minuten unter Rückfluss erhitzt. Nach Abkühlen auf Raumtemperatur wird die Mischung auf Eis/Wasser gegossen, mit Salzsäure neutralisiert. Der ausgefallene Niederschlag wird mit Wasser gewaschen, im Vakuum getrocknet und der trockene Rückstand säulenchromatographisch (Kieselgel-Toluol/Essigsäureethylester 4:1) gereinigt. Smp. 152—154°C. IR (fest/KBr) in $cm^{-1}$: 1655 (CO). $^1H$—NMR ($CDCL_3$) in ppm: 7,0 (breites s, 1H); 7,3 (m, 2H); 9,66 (s, 1H); 11,9 (s, 1H, mit $D_2O$ austauschbares H). Der Massenpeak liegt bei 204. Diese Substanz ist neu, zeigt fungizide Wirkung und ist Bestandteil dieser Erfindung.

b) *Herstellung von 3-Hydroxyiminomethyl-4-(2,3-dichlorphenyl)pyrrol*

5,0 g des nach a) hergestellten 3-Formyl-4-(2,3-dichlorphenyl)pyrrols, 1.7 g Hydroxylamin-hydrochlorid und 2,4 g Natriumacetat werden 3 Stunden bei 80°C in 80 ml Ethanol gerührt. Nach Abkühlen auf Raumtemperatur wird das Reaktionsgemisch auf Eis gegossen, noch 30 Minuten gerührt und der ausgefallene Niederschlag abfiltriert, mit Wasser gewaschen und getrocknet. Man erhält 5,02 g des 3-Hydroxyiminomethyl-4-(2,3-dichlorphenyl)pyrrols als syn/anti-Gemisch. Smp. 158—160°C. Diese Substanz ist ebenfalls neu und zeigt fungizide Wirkung.

c) *Herstellung des Endproduktes 4-(2,3-Dichlorphenyl)-3-cyanopyrrol*

3,2 g des nach b) hergestellten 3-Hydroxyiminomethyl-4-(2,3-dichlorphenyl)pyrrols werden in 50 ml Acetanhydrid 5 Stunden bei ca. 100°C gehalten, anschliessend auf Raumtemperatur abgekühlt, auf Eis/NaOH gegossen und das resultierende Gemisch noch 2 Stunden gerührt. Der ausgefallene Niederschlag wird in Essigsäureethylester gelöst, mit Wasser gewaschen und die Esterphase über Magnesiumsulfat getrocknet. Der eingedampfte Rückstand wird säulenchromatographisch (Kieselgel-Toluol/Essigsäureethylester 4:1) gereinigt. Smp. 149—151°C.

Entsprechend der vorstehend beschriebenen Arbeitsweisen werden auch die nachfolgend genannten Verbindungen der Formel I

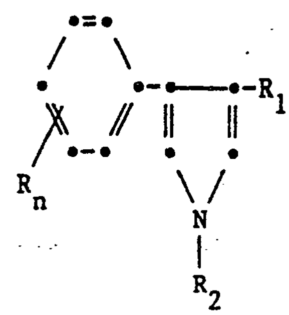

(I)

hergestellt:

Tabelle 4:

| Verb. Nr. | $R_n$ | $R_1$ | $R_2$ | Smp. [°C] |
|---|---|---|---|---|
| 4.1 | H | CN | H | 120-123 |
| 4.2 | 3-Cl | CN | H | 138-140 |
| 4.3 | 2,4-$Cl_2$ | CN | H | 150-152 |
| 4.4 | 4-Cl | CN | H | 153-155 |
| 4.5 | 4-F | CN | H | 137-139 |
| 4.6 | 3-$CH_3$ | CN | H | 109-111 |
| 4.7 | 3-F | CN | H | 138-139 |
| 4.8 | 3-Br | CN | H | 132-134 |

Tabelle 4 (Fortsetzung)

| Verb. Nr. | $R_n$ | $R_1$ | $R_2$ | Smp. [°C] |
|---|---|---|---|---|
| 4.9 | $3\text{-}CF_3$ | CN | H | 87-89 |
| 4.10 | 2-Cl | CN | H | 136-138 |
| 4.11 | $2,3\text{-}Cl_2$ | CN | H | 152-154 |
| 4.12 | $2,5\text{-}Cl_2$ | CN | H | 137-142 |
| 4.13 | 2-Br | CN | H | 135-138 |
| 4.14 | $2,3\text{-}Cl_2$ | $COOCH_3$ | H | 205-206 |
| 4.15 | $2,3\text{-}Cl_2$ | CHO | H | 152-154 |
| 4.16 | 3-Cl | $COOCH_3$ | H | 187-189 |
| 4.17 | $3,4\text{-}Cl_2$ | $COOCH_3$ | H | 183-186 |
| 4.18 | 2-Cl | $COOCH_3$ | H | 198-200 |
| 4.19 | $2,3\text{-}Cl_2$ | $COOC_3H_7\text{-}i$ | H | 153-156 |
| 4.20 | $2,3\text{-}Cl_2$ | $COOC_2H_5$ | H | 149-151 |
| 4.21 | $2,3\text{-}Cl_2$ | CN | $CH_2CH_2CN$ | |
| 4.22 | $2,3\text{-}Cl_2$ | CN | $CH_2CH_2COOCH_3$ | |

Das beschriebene Verfahren ist einschlisselich aller Teilschritte Bestandteile dieser Erfindung.

**Patentansprüche für die Vertragsstaaten: BE CH DE FR GB IT LI LU NL SE**

1. Verfahren zur Herstellung von 4-Phenyl-pyrrol-derivaten der Formel I

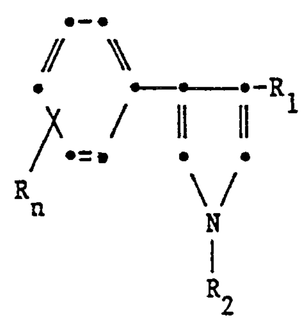

(I)

worin $R_1$ für CN, CHO oder $COOC_1$—$C_6$-Alkyl steht; $R_2$ Wasserstoff, $CH_2CH_2CN$ oder $CH_2CH_2COOC_1$—$C_6$-Alkyl bedeutet; R für Halogen, $C_1$—$C_6$-Alkyl oder $C_1$—$C_6$-Haloalkyl steht; und n für 0, 1 oder 2 steht, dadurch gekennzeichnet, dass man ein Phenacylamin der Formel II

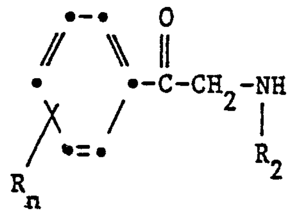

(II)

in Form seines Säureadditionssalzes mit einer Verbindung der Formel III

$$T—CH=CH—R_1 \quad (III)$$

zu einem Zwischenprodukt der Formel IV

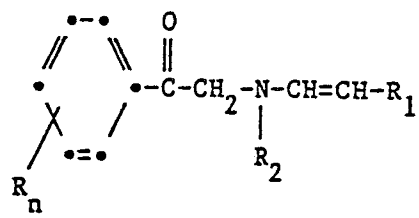

(IV)

umsetzt und dieses in Gegenwart einer Base zu einer Verbindung der Formel I cyclisiert, wobei die Substituenten $R_1$, $R_2$ und $R_n$ in den Formeln II, III und IV die unter Formel I angegebenen Bedeutungen haben, T eine der Gruppe —OZ, —$N(R_3)(R_4)$, —$OCOR_a$, —$OSO_2R_b$, —$SR_c$ oder Halogen repräsentiert, wobei Z für $C_1$—$C_6$-Alkyl, gegebenenfalls substituiertes Phenyl, ein Alkali- oder Erdalkaliatom steht, $R_a$ und $R_b$ unabhängig voneinander für $C_1$—$C_6$-Alkyl oder gegebenenfalls substituiertes Phenyl stehen, $R_c$ für $C_1$—$C_3$-Alkyl, $C_1$—$C_3$-Haloalkyl oder gegebenenfalls substituiertes Phenyl steht; und $R_3$ und $R_4$ unabhängig voneinander für $C_1$—$C_6$-Alkyl stehen oder gemeinsam mit dem Aminstickstoff einen gesättigten fünf- oder sechsgliedrigen Heterocyclus bilden, der als Heteroatom entweder nur den Aminstickstoff oder ein weiteres Heteroatom enthält.

2. Verfahren nach Anspruch 1, dadurch gekennzeichnet, dass man das Phenacylamin der Formel II in Form seines Säureadditionssalzes mit einer Verbindung der Formel III in Gegenwart einer Base direkt zu einem 4-Phenyl-pyrrol-derivat der Formel I cyclisiert.

3. Verfahren nach Anspruch 1, dadurch gekennzeichnet, dass man das Zwischenprodukt der Formel IV zuerst isoliert und dann in Gegenwart einer Base zu einem 4-Phenyl-pyrrol-derivat der Formel I cyclisiert.

4. Verfahren nach einem der Ansprüche 1 bis 3, dadurch gekennzeichnet, dass man ein Ausgangsprodukt der Formel II einsetzt, worin R für Halogen steht, n für 1 oder 2 steht, $R_1$ für CN steht und $R_2$ Wasserstoff bedeutet.

5. Verfahren nach Anspruch 4, dadurch gekennzeichnet, dass in Formel II R für Fluor, Chlor oder Brom steht, n für 2 steht, $R_1$ für CN steht und $R_2$ Wasserstoff bedeutet.

6. Verfahren nach Anspruch 5, dadurch gekennzeichnet, dass in Formel II $R_n$ für 2,3-Dichlor steht, $R_1$ für CN steht und $R_2$ Wasserstoff bedeutet.

7. Verfahren nach einem der Ansprüche 1 bis 6, dadurch gekennzeichnet, dass man die Reaktion in einem reaktionsinerten Lösungsmittel oder Lösungsmittelgemisch durchgeführt.

8. Verfahren nach Anspruch 7, dadurch gekennzeichnet, dass man als Lösungsmittel oder Bestandteil des Lösungsmittelgemisches einen aliphatischen oder aromatischen Kohlenwasserstoff, einen Ether oder eine etherartige Verbindung, ein Nitril, ein N,N-dialkyliertes Amid, Dimethylsulfoxid, ein Keton, einen Alkohol oder Wasser einsetzt.

9. Verfahren nach einem der Ansprüche 1 bis 6, dadurch gekennzeichnet, dass man als Base ein Oxid, Hydrid, Hydroxid, Carbonat, Carbonsäuresalz oder Alkoholat eines Erdalkalimetalls oder Alkalimetals, ein Trialkylamin oder eine Pyridinbase einsetzt.

10. Verfahren nach Anspruch 1 oder 2, dadurch gekennzeichnet, dass man das Phenacylamin II als Säureadditionssalz einer Halogenwasserstoffsäure, der Schwefelsäure oder einer Sulfonsäure einsetzt.

11. Verfahren nach Anspruch 10, dadurch gekennzeichnet, dass man das Phenacylamin der Formel II in Form seines Hydrochlorids oder Hydrobromids einsetzt.

12. Verfahren nach einem der Ansprüche 1 bis 12, dadurch gekennzeichnet, dass man die Umsetzung bei 0° bis +120°C durchführt.

13. Verfahren nach Anspruch 12, dadurch gekennzeichnet, dass man die Umsetzung bei +30° bis +80°C durchführt.

14. Verfahren nach einem der Ansprüche 1 bis 6, dadurch gekennzeichnet, dass man eine Verbindung der Formel III einsetzt, worin T für eine der Gruppen

$-N(CH_3)_2$, $-N(C_2H_5)_2$, 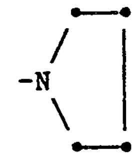, 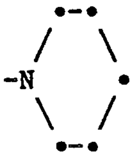, 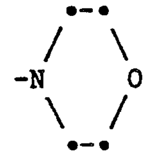, -OK oder -ONa steht.

und $R_1$ für CN, $COOCH_3$ oder CHO steht.

15. Verfahren nach Anspruch 14, dadurch gekennzeichnet, dass T für $—N(CH_3)_2)$ oder $—N(C_2H_5)_2$ steht und $R_1$ CN bedeutet.

16. Verfahren nach einem der Ansprüche 1 bis 3, dadurch gekennzeichnet, dass man bei 0° bis 120°C in Gegenwart eines reaktionsinerten Lösungsmittels 2,3-Dichlorphenacylamin in Form seines Halogenwasserstoffsäureadditionssalzes mit einer Verbindung der Formel III umsetzt, worin $R_1$ für CN steht und T eine der Gruppen

$-N(CH_3)_2$, $-N(C_2H_5)_2$, 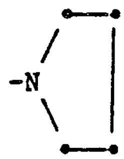, 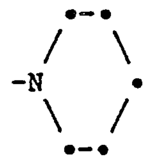, 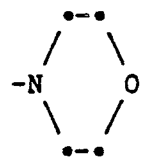, -OK oder -ONa

repräsentiert, zu 3-(2,3-Dichlorphenacylamino)-acrylonitril umsetzt und letzteres als Substanz oder in situ in Gegenwart einer Base zu 4-(2,3-Dichlorphenyl)-3-cyanopyrrol cyclisiert.

17. Verfahren nach einem der Ansprüche 1 oder 16, dadurch gekennzeichnet, dass man die Reaktionspartner in äquimolaren Mengen einsetzt.

18. 2,3-Dichlorphenacylamin unter Einschluss seiner Säureadditionssalze.

19. Verbindungen der Formel IV

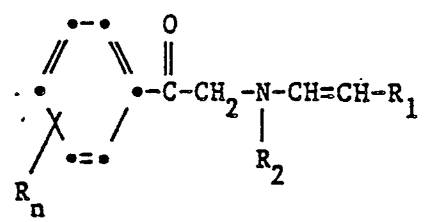

(IV)

worin $R_n$, $R_1$ und $R_2$ die unter Formel I angegebenen Bedeutungen haben.

20. Verbindungen der Formel IV gemäss Anspruch 19, in denen $R_1$ Cyano, $R_2$ Wasserstoff und R Chlor bedeuten und n für 2 steht.

**Patentansprüche für den Vertragsstaat: AT**

1. Verfahren zur Herstellung von 4-Phenyl-pyrrol-derivaten der Formel I

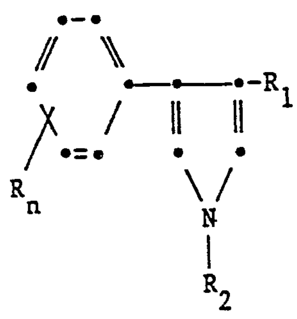

(I)

worin $R_1$ für CN, CHO oder $COOC_1—C_6$-Alkyl steht; $R_2$ Wasserstoff, $CH_2CH_2CN$ oder $CH_2CH_2COOC_1—C_6$-Alkyl bedeutet; R für Halogen, $C_1—C_6$-Alkyl oder $C_1—C_6$-Haloalkyl steht; und n für 0, 1 oder 2 steht, dadurch gekennzeichnet, dass man ein Phenacylamin der Formel II

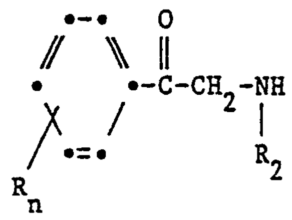

(II)

in Form seines Säureadditionssalzes mit einer Verbindung der Formel III

$$T—CH=CH—R_1 \qquad (III)$$

zu einem Zwischenprodukt der Formel IV

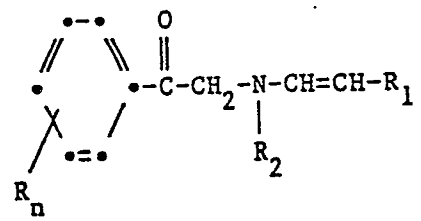

(IV)

umsetzt und dieses in Gegenwart einer Base zu einer Verbindung der Formel I cyclisiert, wobei die Substituenten $R_1$, $R_2$ und $R_n$ in den Formeln II, III und IV die unter Formel I angegebenen Bedeutungen haben, T eine der Gruppe $—OZ$, $—N(R_3)(R_4)$, $—OCOR_a$, $—OSO_2R_b$, $—SR_c$ oder Halogen repräsentiert, wobei Z für $C_1—C_6$-Alkyl, gegebenenfalls substituiertes Phenyl, ein Alkali- oder Erdalkaliatom steht, $R_a$ und $R_b$ unabhängig voneinander für $C_1—C_6$-Alkyl oder gegebenenfalls substituiertes Phenyl stehen, $R_c$ für $C_1—C_3$-Alkyl, $C_1—C_3$-Haloalkyl oder gegebenenfalls substituiertes Phenyl steht; und $R_3$ und $R_4$ unabhängig voneinander für $C_1—C_6$-Alkyl stehen oder gemeinsam mit dem Aminstickstoff einen gesättigten fünf- oder sechsgliedrigen Heterocyclus bilden, der als Heteroatom entweder nur den Aminstickstoff oder ein weiteres Heteroatom enthält.

2. Verfahren nach Anspruch 1, dadurch gekennzeichnet, dass man das Phenacylamin der Formel II in Form seines Säureadditionssalzes mit einer Verbindung der Formel III in Gegenwart einer Base direkt zu einem 4-Phenyl-pyrrol-derivat der Formel I cyclisiert.

3. Verfahren nach Anspruch 1, dadurch gekennzeichnet, dass man das Zwischenprodukt der Formel IV zuerst isoliert und dann in Gegenwart einer Base zu einem 4-Phenyl-pyrrol-derivat der Formel I cyclisiert.

4. Verfahren nach einem der Ansprüche 1 bis 3, dadurch gekennzeichnet, dass man ein Ausgangsprodukt der Formel II einsetzt, worin R für Halogen steht, n für 1 oder 2 steht, $R_1$ für CN steht und $R_2$ Wasserstoff bedeutet.

5. Verfahren nach Anspruch 4, dadurch gekennzeichnet, dass in Formel II R für Fluor, Chlor oder Brom steht, n für 2 steht, $R_1$ für CN steht und $R_2$ Wasserstoff bedeutet.

6. Verfahren nach Anspruch 5, dadurch gekennzeichnet, dass in Formel II $R_n$ für 2,3-Dichlor steht, $R_1$ für CN steht und $R_2$ Wasserstoff bedeutet.

7. Verfahren nach einem der Ansprüche 1 bis 6, dadurch gekennzeichnet, dass man die Reaktion in einem reaktionsinerten Lösungsmittel oder Lösungsmittelgemisch durchgeführt.

8. Verfahren nach Anspruch 7, dadurch gekennzeichnet, dass man als Lösungsmittel oder Bestandteil des Lösungsmittelgemisches einen aliphatischen oder aromatischen Kohlenwasserstoff, einen Ether oder eine etherartige Verbindung, ein Nitril, ein N,N-dialkyliertes Amid, Dimethylsulfoxid, ein Keton, einen Alkohol oder Wasser einsetzt.

9. Verfahren nach einem der Ansprüche 1 bis 6, dadurch gekennzeichnet, dass man als Base ein Oxid, Hydrid, Hydroxid, Carbonat, Carbonsäuresalz oder Alkoholat eines Erdalkalimetalls oder Alkalimetals, ein Trialkylamin oder eine Pyridinbase einsetzt.

10. Verfahren nach Anspruch 1 oder 2, dadurch gekennzeichnet, dass man das Phenacylamin II als Säureadditionssalz einer Halogenwasserstoffsäure, der Schwefelsäure oder einer Sulfonsäure einsetzt.

11. Verfahren nach Anspruch 10, dadurch gekennzeichnet, dass man das Phenacylamin der Formel II in Form seines Hydrochlorids oder Hydrobromids einsetzt.

12. Verfahren nach einem der Ansprüche 1 bis 12, dadurch gekennzeichnet, dass man die Umsetzung bei 0° bis +120°C durchführt.

13. Verfahren nach Anspruch 12, dadurch gekennzeichnet, dass man die Umsetzung bei +30° bis +80°C durchführt.

14. Verfahren nach einem der Ansprüche 1 bis 6, dadurch gekennzeichnet, dass man eine Verbindung der Formel III einsetzt, worin T für eine der Gruppen

$-N(CH_3)_2$, $-N(C_2H_5)_2$, 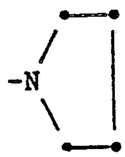, 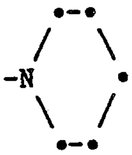, 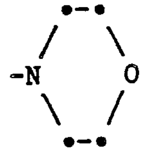, -OK oder -ONa steht

und $R_1$ für CN, $COOCH_3$ oder CHO steht.

15. Verfahren nach Anspruch 14, dadurch gekennzeichnet, dass T für $-N(CH_3)_2$ oder $-N(C_2H_5)_2$ steht und $R_1$ CN bedeutet.

16. Verfahren nach einem der Ansprüche 1 bis 3, dadurch gekennzeichnet, dass man bei 0° bis 120°C in Gegenwart eines reaktionsinerten Lösungsmittels 2,3-Dichlorphenacylamin in Form seines Halogenwasserstoffsäureadditionssalzes mit einer Verbindung der Formel III umsetzt, worin $R_1$ für CN steht und T eine der Gruppen

$-N(CH_3)_2$, $-N(C_2H_5)_2$, 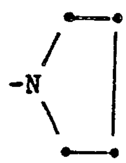, 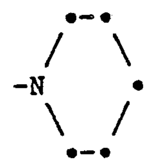, 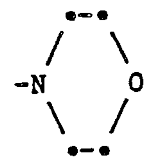, -OK oder -ONa

repräsentiert, zu 3-(2,3-Dichlorphenacylamino)-acrylonitril umsetzt und letzteres als Substanz oder in situ in Gegenwart einer Base zu 4-(2,3-Dichlorphenyl)-3-cyanopyrrol cyclisiert.

17. Verfahren gemäss Anspruch 1 oder 3, dadurch gekennzeichnet, dass als Formel IV eine Verbindung hergestellt wird, in der $R_1$ Cyano, $R_2$ Wasserstoff, R chlor und n 2 bedeuten.

18. Verfahren nach einem der Ansprüche 1 oder 16, dadurch gekennzeichnet, dass man die Reaktionspartner in äquimolaren Mengen einsetzt.

**Revendications pour les Etats contractants: BE CH DE FR GB IT LI LU NL SE**

1. Procédé de préparation de dérivés du 4-phénylpyrrole de formule I

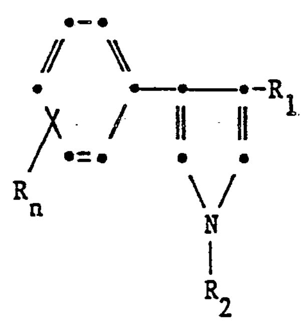

(I)

dans laquelle $R_1$ représente un groupe CN, CHO ou COO-alkyle en $C_1$—$C_6$; $R_2$ représente l'hydrogène, un groupe $CH_2CH_2CN$ ou $CH_2CH_2COO$-alkyle en $C_1$—$C_6$; R représente un halogène, un groupe alkyle en $C_1$—$C_6$ ou halogénoalkyle en $C_1$—$C_6$; et n est égal à 0, 1 ou 2, caractérisé en ce que l'on convertit une phénacylamine de formule II

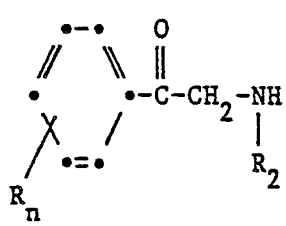

(II)

à l'état de sel formé par addition avec un acide, par réaction avec un composé de formule III

$$T-CH=CH-R_1 \quad (III)$$

en un produit intermédiaire de formule IV

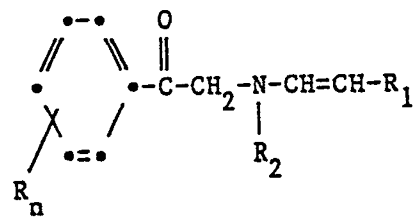

(IV)

qu'on cyclise en présence d'une base, ce qui donne un composé de formule I, les symboles $R_1$, $R_2$ et $R_n$ ayant dans les formules II, III et IV des significations indiquées en référence à la formule I, T représente l'un des groupes —OZ, —$N(R_3)(R_4)$, —$OCOR_a$, —$OSO_2R_b$, —$SR_c$ ou un halogène, Z représente un groupe alkyle en $C_1$—$C_6$, phényle éventuellement substitué, un atome de métal alcalin ou alcalino-terreux, $R_a$ et $R_b$ représentent chacun, indépendamment l'un de l'autre, un groupe alkyle en $C_1$—$C_6$ ou un groupe phényle éventuellement substitué, $R_c$ représente un groupe alkyle en $C_1$—$C_3$, halogénoalkyle en $C_1$—$C_3$ ou phényle éventuellement substitué; et $R_3$ et $R_4$ représentent chacun, indépendamment l'un de l'autre, un groupe alkyle en $C_1$—$C_6$ ou forment ensemble et avec l'atome d'azote aminé un hétérocycle saturé à 5 ou 6 chaînons contenant en tant qu'hétéroatome uniquement l'azote aminé ou contenant encore un autre hétéroatome.

2. Procédé selon la revendication 1, caractérisé en ce que l'on cyclise directement la phénacylamine de formule II, à l'état de sel formé par addition avec un acide, avec un composé de formule III, en présence d'une base, en un dérivé du 4-phényl-pyrrole de formule I.

3. Procédé selon la revendication 1, caractérisé en ce que l'on isole d'abord le produit intermédiaire de formule IV qu'on cyclise ensuite en présence d'une base en un dérivé du 4-phényl-pyrrole de formule I.

4. Procédé selon l'une des revendications 1 à 3, caractérisé en ce que l'on part d'un composé de formule II dans laquelle R représente un halogène, n est égal à 1 ou 2, $R_1$ représente un groupe CN et $R_2$ l'hydrogène.

5. Procédé selon la revendication 4, caractérisé en ce que, dans la formule II, R représente le fluor, le chlore ou le brome, n est égal à 2, $R_1$ représente un groupe CN et $R_2$ l'hydrogène.

6. Procédé selon la revendication 5, caractérisé en ce que, dans la formule II, $R_n$ représente un substituant 2,3-dichloro, $R_1$ représente un groupe CN et $R_2$ l'hydrogène.

7. Procédé selon l'une des revendications 1 à 6, caractérisé en ce que l'on effectue la réaction dans un solvant ou mélange solvant inerte dans la réaction.

8. Procédé selon la revendication 7, caractérisé en ce que l'on utilise en tant que solvant ou composant du mélange solvant un hydrocarbure aliphatique ou aromatique, un éther ou composé apparenté, un nitrile, un N,N-dialkylamide, le diméthylsulfoxyde, une cétone, un alcool ou l'eau.

9. Procédé selon l'une des revendications 1 à 6, caractérisé en ce que l'on utilise en tant que base un oxyde, hydrure, hydroxyde, carbonate, carboxylate ou alcoolate d'un métal alcalino-terreux ou alcalin, une trialkylamine ou une base pyridique.

10. Procédé selon la revendication 1 ou 2, caractérisé en ce que la phénacylamine II est mise en oeuvre à l'état de sel formé par addition avec un hydracide halogéné, l'acide sulfurique ou un acide sulfonique.

11. Procédé selon la revendication 10, caractérisé en ce que la phénacylamine de formule II est mise en oeuvre à l'état de chlorhydrate ou de bromhydrate.

12. Procédé selon l'une des revendications 1 à 11, caractérisé en ce que la réaction est effectuée à des températures de 0 à +120°C.

13. Procédé selon la revendication 12, caractérisé en ce que la réaction est effectuée à des températures de +30 à +80°C.

14. Procédé selon l'une des revendications 1 à 6, caractérisé en ce que l'on utilise un composé de formule III dans laquelle T représente l'un des groupes —$N(CH_3)_2$,

$-N(C_2H_5)_2$,

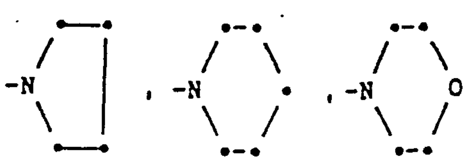

, -OK ou -ONa

et $R_1$ représente un groupe CN, $COOCH_3$ ou CHO.

15. Procédé selon la revendication 14, caractérisé en ce que T représente —$N(CH_3)_2$ ou —$N(C_2H_5)_2$ et $R_1$ un groupe CN.

16. Procédé selon l'une des revendications 1 à 3, caractérisé en ce que l'on fait réagir la 2,3-dichlorophénacylamine à l'état de sel formé par addition avec un hydracide halogéné à des températures de 0 à 120°C en présence d'un solvant inerte avec un composé de formule III dans laquelle $R_1$ représente CN et T l'un des groupes

$-N(CH_3)_2$, $-N(C_2H_5)_2$, 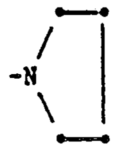, 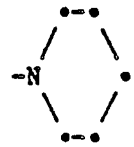, 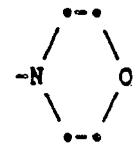, —OK ou —ONa,

ce qui donne le 3-(2,3-dichlorophénacylamino)-acrylonitrile qu'on cyclise après l'avoir isolé ou in situ en présence d'une base, en le 4-(2,3-dichlorophényl)-3-cyanopyrrole.

17. Procédé selon l'une des revendications 1 ou 16, caractérisé en ce que l'on met les réactifs en oeuvre en quantités équimoléculaires.

18. La 2,3-dichlorophénacylamine, y compris ses sels formés par addition avec des acides.

19. Composés de formule IV

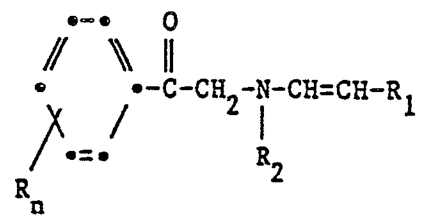

(IV)

dans laquelle $R_n$, $R_1$ et $R_2$ ont les significations indiquées en référence à la formule I.

20. Composés de formule IV selon la revendication 19, dans lesquels $R_1$ représente un groupe cyano, $R_2$ l'hydrogène et R le chloro, et n est égal à 2.

**Revendications pour l'Etat contractant: AT**

1. Procédé de préparation de dérivés du 4-phényl-pyrrole de formule I

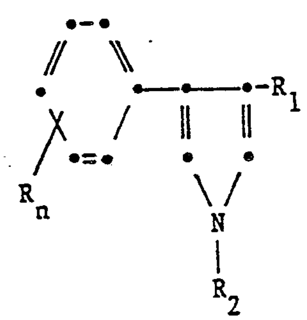

(I)

dans laquelle $R_1$ représente un groupe CN, CHO ou COO-alkyle en $C_1$—$C_6$; $R_2$ représente l'hydrogène, un groupe $CH_2CH_2CN$ ou $CH_2CH_2COO$-alkyle en $C_1$—$C_6$; R représente un halogène, un groupe alkyle en $C_1$—$C_6$ ou halogénoalkyle en $C_1$—$C_6$; et n est égal à 0, 1 ou 2, caractérisé en ce que l'on convertit une phénacylamine de formule II

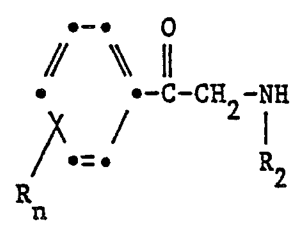

(II)

à l'état de sel formé par addition avec un acide, par réaction avec un composé de formule III

$$T—CH=CH—R_1 \qquad (III)$$

en un produit intermédiaire de formule IV

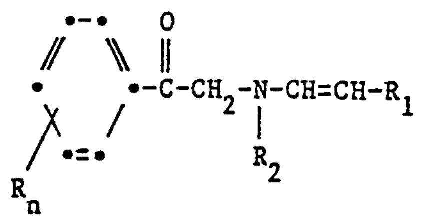

(IV)

qu'on cyclise en présence d'une base, ce qui donne un composé de formule I, les symboles $R_1$, $R_2$ et $R_n$ ayant dans les formules II, III et IV des significations indiquées en référence à la formule I, T représente l'un des groupes —OZ, —$N(R_3)(R_4)$, —$OCOR_a$, —$OSO_2R_b$, —$SR_c$ ou un halogène, Z représente un groupe alkyle en $C_1$—$C_6$, phényle éventuellement substitué, un atome de métal alcalin ou alcalino-terreux, $R_a$ et $R_b$ représentent chacun, indépendamment l'un de l'autre, un groupe alkyle en $C_1$—$C_6$ ou un groupe phényle éventuellement substitué, $R_c$ représente un groupe alkyle en $C_1$—$C_3$, halogénoalkyle en $C_1$—$C_3$ ou phényle éventuellement substitué; et $R_3$ et $R_4$ représentent chacun, indépendamment l'un de l'autre, un groupe alkyle en $C_1$—$C_6$ ou forment ensemble et avec l'atome d'azote aminé un hétérocycle saturé à 5 ou 6 chaînons contenant en tant qu'hétéroatome uniquement l'azote aminé ou contenant encore un autre hétéroatome.

2. Procédé selon la revendication 1, caractérisé en ce que l'on cyclise directement la phénacylamine de formule II, à l'état de sel formé par addition avec un acide, avec un composé de formule III, en présence d'une base, en un dérivé du 4-phényl-pyrrole de formule I.

3. Procédé selon la revendication 1, caractérisé en ce que l'on isole d'abord le produit intermédiaire de formule IV qu'on cyclise ensuite en présence d'une base en un dérivé du 4-phényl-pyrrole de formule I.

4. Procédé selon l'une des revendications 1 à 3, caractérisé en ce que l'on part d'un composé de formule II dans laquelle R représente un halogène, n est égal à 1 ou 2, $R_1$ représente un groupe CN et $R_2$ l'hydrogène.

5. Procédé selon la revendication 4, caractérisé en ce que, dans la formule II, R représente le fluor, le chlore ou le brome, n est égal à 2, $R_1$ représente un groupe CN et $R_2$ l'hydrogène.

6. Procédé selon la revendication 5, caractérisé en ce que, dans la formule II, $R_n$ représente un substituant 2,3-dichloro, $R_1$ représente un groupe CN et $R_2$ l'hydrogène.

7. Procédé selon l'une des revendications 1 à 6, caractérisé en ce que l'on effectue la réaction dans un solvant ou mélange solvant inerte dans la réaction.

8. Procédé selon la revendication 7, caractérisé en ce que l'on utilise en tant que solvant ou composant du mélange solvant un hydrocarbure aliphatique ou aromatique, un éther ou composé apparenté, un nitrile, un N,N-dialkylamide, le diméthylsulfoxyde, une cétone, un alcool ou l'eau.

9. Procédé selon l'une des revendications 1 à 6, caractérisé en ce que l'on utilise en tant que base un oxyde, hydrure, hydroxyde, carbonate, carboxylate ou alcoolate d'un métal alcalino-terreux ou alcalin, une trialkylamine ou une base pyridique.

10. Procédé selon la revendication 1 ou 2, caractérisé en ce que la phénacylamine II est mise en oeuvre à l'état de sel formé par addition avec un hydracide halogéné, l'acide sulfurique ou un acide sulfonique.

11. Procédé selon la revendication 10, caractérisé en ce que la phénacylamine de formule II est mise en oeuvre à l'état de chlorhydrate ou de bromhydrate.

12. Procédé selon l'une des revendications 1 à 11, caractérisé en ce que la réaction est effectuée à des températures de 0 à +120°C.

13. Procédé selon la revendication 12, caractérisé en ce que la réaction est effectuée à des températures de +30 à +80°C.

14. Procédé selon l'une des revendications 1 à 6, caractérisé en ce que l'on utilise un composé de formule III dans laquelle T représente l'un des groupes

$-N(CH_3)_2$, $-N(C_2H_5)_2$, 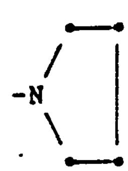, 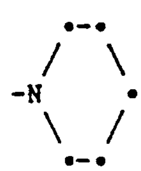, 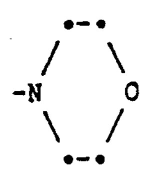, -OK ou -ONa

et $R_1$ représente un groupe CN, $COOCH_3$ ou CHO.

15. Procédé selon la revendication 14, caractérisé en ce que T représente —$N(CH_3)_2$ ou —$N(C_2H_5)_2$ et $R_1$ un groupe CN.

16. Procédé selon l'une des revendications 1 à 3, caractérisé en ce que l'on fait réagir la 2,3-dichlorophénacylamine à l'état de sel formé par addition avec un hydracide halogéné à des températures de 0 à 120°C en présence d'un solvant inerte avec un composé de formule III dans laquelle $R_1$ représente CN et T l'un des groupes

$-N(CH_3)_2$, $-N(C_2H_5)_2$, 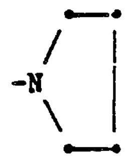 , 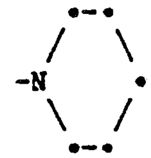 , 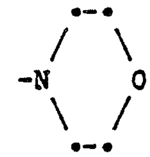 , —OK ou —ONa,

ce qui donne le 3-(2,3-dichlorophénacylamino)-acrylonitrile qu'on cyclise après l'avoir isolé ou in situ en présence d'une base, en le 4-(2,3-dichlorophényl)-3-cyanopyrrole.

17. Procédé selon la revendication 1 ou 3, caractérisé en ce que l'on utilise un composé de formule IV dans laquelle $R_1$ représente un groupe cyano, $R_2$ l'hydrogène, R le chlore et n est égal à 2.

18. Procédé selon l'une des revendications 1 ou 16, caractérisé en ce que l'on met les réactifs en oeuvre en quantités équimoléculaires.

**Claims for the Contracting States: BE CH DE FR GB IT LI LU NL SE**

1. A process for the preparation of a 4-phenylpyrrole derivative of the formula I

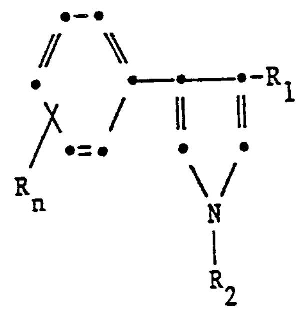

(I)

in which $R_1$ is CN, CHO or $COOC_1$—$C_6$alkyl; $R_2$ is hydrogen, $CH_2CH_2CN$ or $CH_2CH_2COOC_1$—$C_6$alkyl; R is halogen, $C_1$—$C_6$alkyl or $C_1$—$C_6$haloalkyl; and n is 0, 1 or 2, which comprises reacting a phenacylamine of the formula II

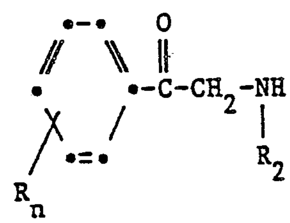

(II)

in the form of an acid addition salt, with a compound of the formula III

$$T—CH=CH—R_1 \qquad (III)$$

to give an intermediate of the formula IV

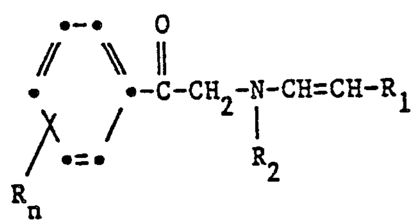

(IV)

and cyclising this compound in the presence of a base, to a compound of the formula I, in which formulae II, III and IV the substituents $R_1$, $R_2$ and $R_n$ are as defined for formula I, T is a group selected from —OZ, —$N(R_3)(R_4)$, —$OCOR_a$, —$OSO_2R_b$, —$SR_c$ or halogen, where Z is $C_1$—$C_6$alkyl, unsubstituted or substituted phenyl, an alkali metal atom or an alkaline earth metal atom, each of $R_a$ and $R_b$ independently of the other is $C_1$—$C_6$alkyl or unsubstituted or substituted phenyl, $R_c$ is $C_1$—$C_3$alkyl, $C_1$—$C_3$haloalkyl or unsubstituted or substituted phenyl; and each of $R_3$ and $R_4$ independently of the other is $C_1$—$C_6$alkyl or, together with the amine nitrogen, form a saturated 5- or 6-membered heterocyclic ring which contains, as hetero atom, either

only the amine nitrogen or a further hetero atom.

2. A process according to claim 1, which comprises cyclising the phenacylamine of the formula II, in the form of an acid addition salt, with a compound of the formula III, in the presence of a base, directly to a 4-phenylpyrrole derivative of the formula I.

3. A process according to claim 1, which comprises first isolating the intermediate of the formula IV, and then cyclising it in the presence of a base, to a 4-phenylpyrrole derivative of the formula I.

4. A process according to any one of claims 1 to 3, which comprises using a starting material of the formula II, in which R is halogen, n is 1 or 2, $R_1$ is CN and $R_2$ is hydrogen.

5. A process according to claim 4, wherein in formula II R is fluorine, chlorine or bromine, n is 2, $R_1$ is CN and $R_2$ is hydrogen.

6. A process according to claim 5, wherein in formula II $R_n$ is 2,3-dichloro, $R_1$ is CN and $R_2$ is hydrogen.

7. A process according to any one of claims 1 to 6, wherein the reaction is carried out in an inert solvent or mixture of solvents.

8. A process according to claim 7, wherein the solvent or component of the solvent mixture used is an aliphatic or aromatic hydrocarbon, an ether or an ethereal compound, a nitrile, an N,N-dialkylated amide, dimethylsulfoxide, a ketone, an alcohol or water.

9. A process according to any one of claims 1 to 6, wherein the base used is an oxide, hydride, hydroxide, carbonate, carboxylic acid salt or alcoholate of an alkaline earth metal or of an alkali metal, a trialkylamine or a pyridine base.

10. A process according to either of claims 1 or 2, wherein the phenacylamine II is employed as acid addition salt of a hydrohalic acid, of sulfuric acid or of a sulfonic acid.

11. A process according to claim 10, wherein the phenacylamine of the formula II is employed in the form of its hydrochloride or hydrobromide.

12. A process according to any one of claims 1 to 12, wherein the reaction is carried out at 0° to +120°C.

13. A process according to claim 12, wherein the reaction is carried out at +30° to +80°C.

14. A process according to any one of claims 1 to 6, which comprises using a compound of the formula III, in which T is one of the groups

$N(CH_3)_2$, $-N(C_2H_5)_2$, 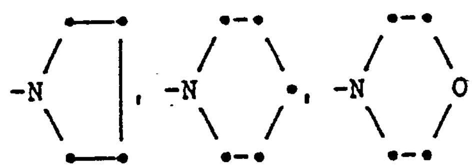 , -OK or -ONa,

and $R_1$ is CN, $COOCH_3$ or CHO.

15. A process according to claim 14, wherein T is $-N(CH_3)_2$ or $-N(C_2H_5)_2$ and $R_1$ is CN.

16. A process according to any one of claims 1 to 3, which comprises reacting 2,3-dichlorophenacylamine in the form of its hydrohalic acid addition salt, at 0° to 120°C and in the presence of an inert solvent, with a compound of the formula III, in which $R_1$ is CN and T is one of the groups

$-N(CH_3)_2$, $-N(C_2H_5)_2$, 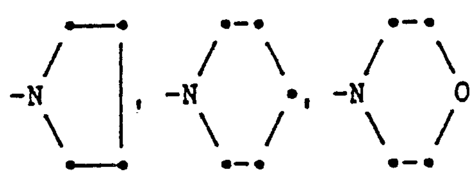 , -OK or -ONa,

to give 3-(2,3-dichlorophenacylamino)-acrylonitrile, and cyclising this compound in substance or in situ, in the presence of a base, to give 4-(2,3-dichlorophenyl)-3-cyanopyrrole.

17. A process according to either of claims 1 or 16, wherein the reactants are employed in equimolar amounts.

18. 2,3-Dichlorophenacylamine or an acid addition salt thereof.

19. A compound of the formula IV

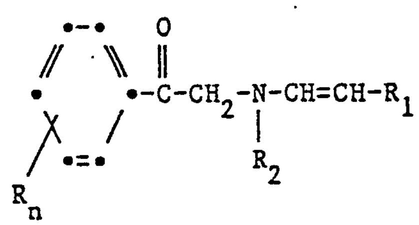

(IV)

in which $R_n$, $R_1$ and $R_2$ are as defined for formula I.

20. A compound of the formula IV according to claim 19, in which $R_1$ is cyano, $R_2$ is hydrogen, R is chlorine and n is 2.

**Claims for the Contracting State: AT**

1. A process for the preparation of a 4-phenylpyrrole derivative of the formula I

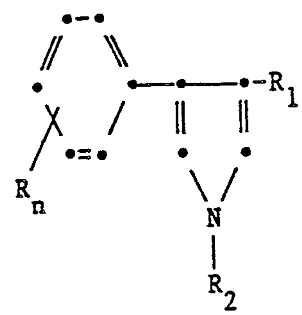

(I)

in which $R_1$ is CN, CHO or $COOC_1$—$C_6$alkyl; $R_2$ is hydrogen, $CH_2CH_2CN$ or $CH_2CH_2COOC_1$—$C_6$alkyl; R is halogen, $C_1$—$C_6$alkyl or $C_1$—$C_6$haloalkyl; and n is 0, 1 or 2, which comprises reacting a phenacylamine of the formula II

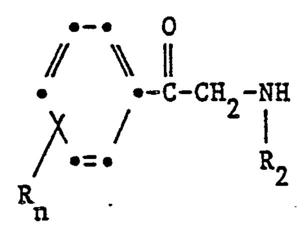

(II)

in the form of an acid addition salt, with a compound of the formula III

$$T—CH=CH—R_1 \qquad (III)$$

to give an intermediate of the formula IV

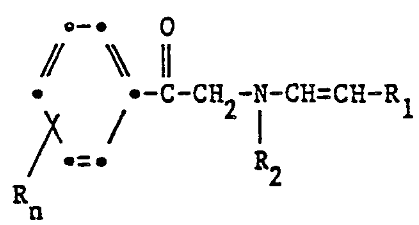

(IV)

and cyclising this compound in the presence of a base, to a compound of the formula I, in which formulae II, III and IV the substituents $R_1$, $R_2$ and $R_n$ are as defined for formula I, T is a group selected from —OZ, —$N(R_3)(R_4)$, —$OCOR_a$, —$OSO_2R_b$, —$SR_c$ or halogen, where Z is $C_1$—$C_6$alkyl, unsubstituted or substituted phenyl, an alkali metal atom or an alkaline earth metal atom, each of $R_a$ and $R_b$ independently of the other is $C_1$—$C_6$alkyl or unsubstituted or substituted phenyl, $R_c$ is $C_1$—$C_3$alkyl, $C_1$—$C_3$haloalkyl or unsubstituted or substituted phenyl; and each of $R_3$ and $R_4$ independently of the other is $C_1$—$C_6$alkyl or, together with the amine nitrogen, form a saturated 5- or 6-membered heterocyclic ring which contains, as hetero atom, either only the amine nitrogen or a further hetero atom.

2. A process according to claim 1, which comprises cyclising the phenacylamine of the formula II, in the form of an acid addition salt, with a compound of the formula III, in the presence of a base, directly to a 4-phenylpyrrole derivative of the formula I.

3. A process according to claim 1, which comprises first isolating the intermediate of the formula IV, and then cyclising it in the presence of a base, to a 4-phenylpyrrole derivative of the formula I.

4. A process according to any one of claims 1 to 3, which comprises using a starting material of the formula II, in which R is halogen, n is 1 or 2, $R_1$ is CN and $R_2$ is hydrogen.

5. A process according to claim 4, wherein in formula II R is fluorine, chlorine or bromine, n is 2, $R_1$ is CN and $R_2$ is hydrogen.

6. A process according to claim 5, wherein in formula II $R_n$ is 2,3-dichloro, $R_1$ is CN and $R_2$ is hydrogen.

7. A process according to any one of claims 1 to 6, wherein the reaction is carried out in an inert solvent or mixture of solvents.

8. A process according to claim 7, wherein the solvent or component of the solvent mixture used is an aliphatic or aromatic hydrocarbon, an ether or an ethereal compound, a nitrile, an N,N-dialkylated amide, dimethylsulfoxide, a ketone, an alcohol or water.

9. A process according to any one of claims 1 to 6, wherein the base used is an oxide, hydride, hydroxide, carbonate, carboxylic acid salt or alcoholate of an alkaline earth metal or of an alkali metal, a trialkylamine or a pyridine base.

10. A process according to either of claims 1 or 2, wherein the phenacylamine II is employed as acid addition salt of a hydrohalic acid, of sulfuric acid or of a sulfonic acid.

11. A process according to claim 10, wherein the phenacylamine of the formula II is employed in the form of its hydrochloride or hydrobromide.

12. A process according to any one of claims 1 to 12, wherein the reaction is carried out at 0° to +120°C.

13. A process according to claim 12, wherein the reaction is carried out at +30° to +80°C.

14. A process according to any one of claims 1 to 6, which comprises using a compound of the formula III, in which T is one of the groups

$N(CH_3)_2$, $-N(C_2H_5)_2$, 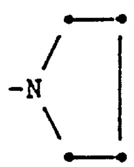 , 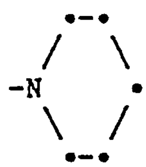 , 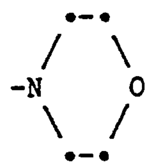 , -OK or -ONa,

and $R_1$ is CN, $COOCH_3$ or CHO.

15. A process according to claim 14, wherein T is $—N(CH_3)_2$ or $—N(C_2H_5)_2$ and $R_1$ is CN.

16. A process according to any one of claims 1 to 3, which comprises reacting 2,3-dichlorophenacylamine in the form of its hydrohalic acid addition salt, at 0° to 120°C and in the presence of an inert solvent, with a compound of the formula III, in which $R_1$ is CN and T is one of the groups

$-N(CH_3)_2$, $-N(C_2H_5)_2$, $-N(C_2H_5)_2$, 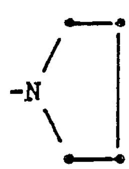 , 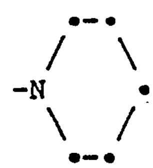 , 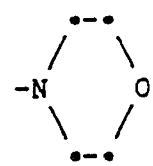 .

17. A process according to either of claims 1 or 3, wherein as formula IV a compound is prepared in which $R_1$ is cyano, $R_2$ is hydrogen, R is chlorine and n is 2.

18. A process according to either of claims 1 or 16, wherein the reactants are employed in equimolar amounts.